Europäisches Patentamt

European Patent Office

Office européen des brevets

(11) Veröffentlichungsnummer: **0 531 717 A2**

# (12) EUROPÄISCHE PATENTANMELDUNG

(21) Anmeldenummer: **92113466.4**

(22) Anmeldetag: **07.08.92**

(51) Int. Cl.5: **C12N 15/76**, C12N 15/70

(30) Priorität: **09.08.91 DE 4126415**

(43) Veröffentlichungstag der Anmeldung:
**17.03.93 Patentblatt 93/11**

(84) Benannte Vertragsstaaten:
**AT BE CH DE DK ES FR GB GR IT LI LU NL SE**

(71) Anmelder: **HOECHST AKTIENGESELLSCHAFT**
**Postfach 80 03 20**
**W-6230 Frankfurt am Main 80(DE)**

(72) Erfinder: **Labes, Gabriele**
**Meller Strasse 41**
**W-4800 Bielefeld(DE)**
Erfinder: **Wohlleben, Wolfgang, Dr.**
**Dürerstrasse 46**
**W-4800 Bielefeld(DE)**

(54) **Ein Promoter-Suchvekfor, damit gefundene Streptomycetenpromotoren sowie deren Isolierung und Verwendung.**

(57) Die Erfindung betrifft einen Promotor-Suchvektor, Verfahren zur Identifizierung und Isolierung von Streptomyceten-Promotoren mit Hilfe dieses Suchvektors sowie die isolierten Promotoren selbst, vorzugsweise die nachstehend beschriebenen Promotoren pS1 und p14 des S. ghanaensis Phagen I19.

Fig. 1

mcs: EcoRI SstI KpnI SmaI PstI HindIII ClaI XbaI BamHI EcoRI SstI KpnI

Die Erfindung betrifft einen Promotor-Suchvektor, Verfahren zur Identifizierung und Isolierung von Streptomyceten-Promotoren mit Hilfe dieses Suchvektors sowie die isolierten Promotoren selbst, vorzugsweise die nachstehend beschriebenen Promotoren pS1 und p14 des S. ghanaensis Phagen I19.

Für die homologe und heterologe Genexpression in Streptomyceten stehen bislang nur relativ wenige starke Promotoren zur Verfügung. Die bislang zu diesem Zweck eingesetzten und beschriebenen Promotoren entstammen hauptsächlich Antibiotikaresistenzgenen als auch anderen Genen, deren Expression durch andere Genprodukte reguliert wird.

Um Streptomyceten als Wirtsorganismus zur starken Expression beliebiger Gene besser nutzen zu können, werden entsprechend stark konstitutive Promotoren als auch Promotoren definierter Stärke benötigt. Solche Promotoren kann man z.B. in der DNA lytischer Bakteriophagen finden.

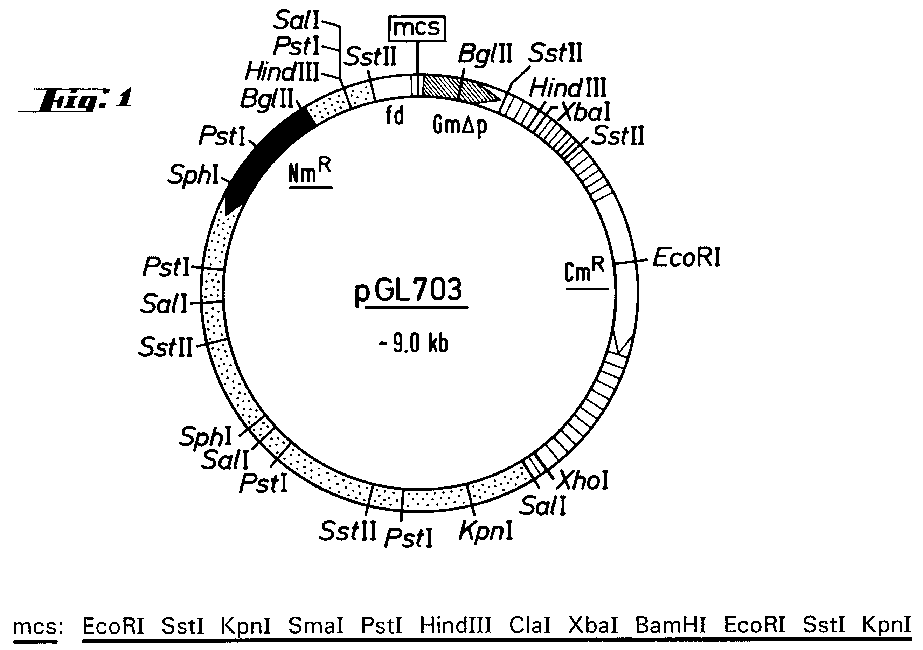

Mit Hilfe des Promotorsuchvektors pGL703 (Fig. 1) konnten geeignete Phagen-Promotoren isoliert werden.

Bei diesem Suchvektor handelt es sich um ein Shuttle-Plasmid, das die Replikationsregionen des E. coli Plasmides pACYC184 und des Streptomyceten-Plasmides pSG5 trägt. Als Selektionsmarker für Streptomyceten besitzt es das Neomycin-Resistenzgen (NmR) von Tn5, als neues Indikatorgen das promotorlose Gentamicinresistenzgen von Tn1696 (GmΔp). Zusätzlich weist es das Chloramphenicol-Resistenzgen (CmR) als E. coli Marker auf. Stromaufwärts des Indikatorgens befindet sich der Transkriptionsterminator des Phagen fd und eine mehrfache Klonierungsstelle (mcs), die u.a. eine singuläre BamHI- und ClaI-Schnittstelle aufweist. Beide Restriktionsstellen können für die Shot-gun Klonierung von DNA verwendet werden, welche mit den Vielschneiderenzymen Sau3A bzw. TaqI bei entsprechend modifizierter DNA restringiert wurde.

Das promotorlose Gentamicin-Resistenzgen als wesentlicher Bestandteil besitzt im 5'-Kodierbereich zwei TTA-Kodons, die in konstitutiv exprimierten Streptomyceten-Genen nicht bzw. sehr selten vorkommen. Eine entsprechende Expression des Gens, nachweislich durch eine vermittelnde Resistenz, findet daher nur bei einem entsprechend hohen m-RNA Spiegel in der Zelle statt. Aus diesem Grund ist das Indikatorgen besonders gut für die Identifizierung stärkerer Promotoren geeignet. Ein weiterer Vorteil des Suchvektors ist die Möglichkeit, Promotor-tragende DNA-Fragmente auf ihre Aktivität hin direkt, d.h. ohne Umklonierungen, in E. coli zu testen.

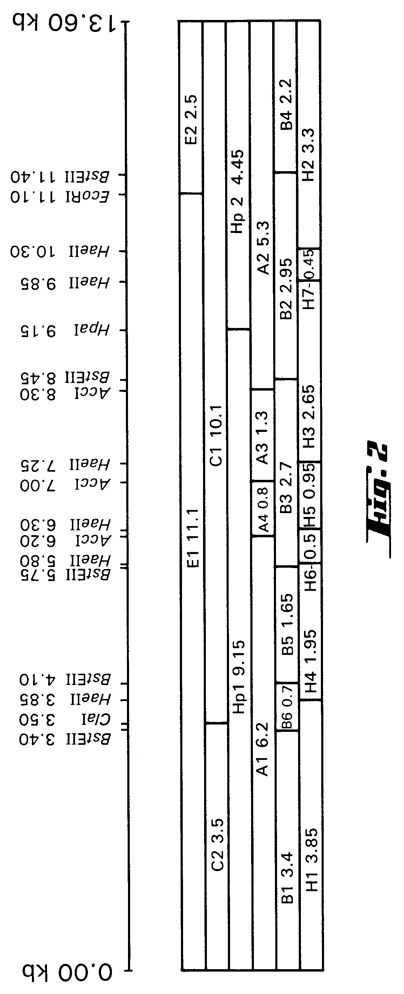

Als sehr geeigneter Spenderorganismus für Promotoren hat sich der virulente S. ghanaensis Phage I19 erwiesen, der mit 13.6kb das kleinste aller bisher beschriebenen Actinophagen-Genome besitzt (Fig. 2).

Die DNA des I19 Phagen liegt modifiziert vor, so daß sie nicht mit dem gängigen Vielschneiderenzym Sau3A subklonierbar ist. Hier kommt eine weitere Eignung des Suchvektors zur Geltung, da er neben einer singulären BamHI- eine singuläre ClaI-Schnittstelle vor dem Indikatorgen aufweist, welche die Subklonierung der Phagen-DNA mittels des alternativen Vielschneiderenzyms TaqI erlaubt. Plasmide mit Promotor-tragenden DNA-Fragmenten können durch Primärselektion der Transformanten auf Gentamicin-haltigem Medium identifiziert und zusätzlich auf die Expression des Neomycin-Resistenzgens hin getestet werden. Die Höhe der Gentamicinresistenz als auch die von den isolierten Promotoren aus gebildete Transkriptmenge sind ein Maß für die Promotorstärke.

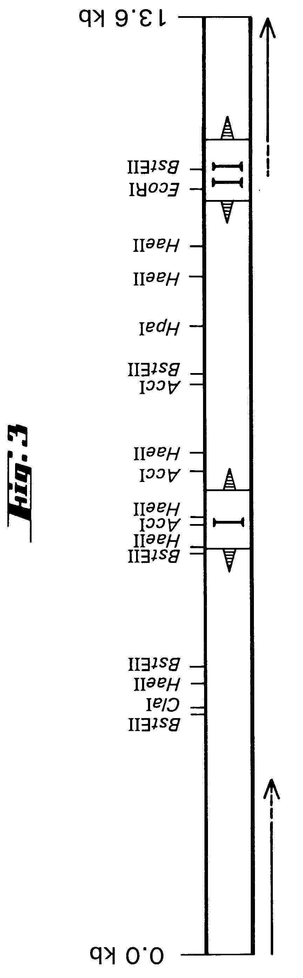

Es konnten zwei Promotor-tragende Regionen auf dem Phagen-Genom identifiziert werden (Fig. 3). Aus ihnen stammen u.a. die Promotoren p14 und pS1, die unterschiedliche Transkriptionsaktivitäten aufweisen. Die Sequenzdaten dieser Promotoren sind in Tab. 1 und Tab. 2 aufgeführt.

Der Promotor p14 führt in Tests zur Bestimmung der minimalen Inhibitionskonzentration (MIC) zu einer etwa doppelt so starken Genexpression wie der erm-up Promotor (Bibb MJ, Janssen GR (1986) Unusual features of transcription and translation of antibiotic resistance genes in antibiotic-producing Streptomyces. Fifth International Symposium on the Genetics of Industrial Microorganisms, 1986, eds.: Alacevic M, Hranueli D, Toman Z), der zu den bislang stärksten Streptomyceten-Promotoren zählt. Im Gegensatz zu diesem ist p14 auch in E. coli aktiv. Entsprechende MIC-Teste zeigen im Vergleich zu dem starken synthetischen Hybridpromotor tac eine dreifach stärkere Expressionsrate. Es können E. coli ähnliche -10 und -35 Konsensusregionen (I;II) identifiziert werden, die aber im Vergleich zu den bisher beschriebenen SEP-Sequenzen neuartig strukturiert sind. Die Promotorregionen sind nicht tandemartig angeordnet, sondern sind direkt benachbart (-35, I,II) bzw. teilweise überlappend (-10, I,II). Der Abstand beider Regionen entspricht 17 (I) bzw. 19 (II) Basenpaare und liegt somit im Optimalbereich. Während die -35 Regionen bis auf ein (II) bzw. zwei (I) Basenpaare der aufgestellten SEP-Konsensussequenz (-TTGaca-) entsprechen, weichen die -10 Regionen stärker von der jeweiligen Konsensussequenz ab. Hier findet man das Motiv ATCAAT (I) bzw. TACAAT (II), wobei den vier Basen CAAT eine essentielle Rolle zugeordnet wird. Kurze direkte Sequenzwiederholungen als auch ein langer Repeat von 9 Basenpaaren (siehe 1 und 2 in Tab. 1) sowie zwei potentielle Ribosomenbindungsstellen (RBS I, II) sind ebenfalls kennzeichnend für den Promotorbereich.

Der Promotor pS1, der nicht in E. coli aktiv ist, weist ebenfalls kurze Sequenzwiederholungen auf 1,1',2, wobei 2 Bestandteil einer sechs Basenpaare langen Duplikation sind. In dieser befindet sich das Motif - CAGAAG -, welches möglicherweise eine Funktion bei der RNA-Polymerase-Erkennung besitzt im Sinn der konventionellen -10 Promotorregionen (I). Stromaufwärts der ersten potentiellen -10 Region (I) befindet sich eine -35 ähnliche Sequenz (II). Ferner ist eine sehr gute Ribosomenbindungsstelle (RBS) identifizierbar. Bezüglich der Gentamicin-Expressionsrate ist pS1 mit p14 vergleichbar, während er im Neomycinresistenztest nur etwa ein Viertel der Aktivität des erm-up Promotor (Bibb M.J.; Janssen G.R; Unusual features of transcription and translation of antibiotic resistance genes in antibiotic-producing Streptomyces; in: Fifth International Symposium on the Genetics of Industrial Microorganisms, 1986, eds.: Alacevic M.; Hranueli D.; Toman Z.) aufweist. Er ist aus Gründen der biologischen Sicherheit einsetzbar, da nur im Streptomyceten aktiv.

Die Erfindung betrifft folglich den Promotorsuchvektor pGL 703 sowie analog aufgebaute Vektoren, die das promotorlose Gentamicin-Resistenzgen enthalten, Verfahren zur Isolierung von Promotoren mit Hilfe solcher Vektoren, sowie die damit gefundenen Promotoren, vorzugsweise p14 und pS1 und schließlich deren Verwendung. In den nachfolgenden Beispielen ist die Erfindung weiter erläutert.

Beispiel 1: Charakterisierung der I19 DNA

Der Phage I19 wurde aus dem Bakterium S. ghanaensis als lytischer Phage (Plaques mit einem Durchmesser von 1.5-2.0 mm) isoliert und charakterisiert. Während der Durchmesser des Phagenkopfes (82,8 nm) und die Schwanzlänge (371,8 nm) sich im Bereich von bisher beschriebenen Bakteriophagen befinden, ist die I19 DNA mit 13.6 kb die kleinste aller bislang bekannten Actinophagen-DNA's. Die Restriktionsanalyse (Fig. 2) ergab, daß die DNA nicht mit den Enzymen BamHI und Sau3A spaltbar ist und somit modifiziert vorliegt. Sie liegt als doppelsträngiges, lineares Molekül im Phagenkopf verpackt vor. Es konnten keine kohäsiven aber homologe Enden nachgewiesen werden. Das Phagen-Genom besitzt zwei Promotorregionen (Fig. 3, I und II), wobei sich der starke p14 Promotor in Region II befindet.

Beispiel 2: Identifizierung von I19 Phagenpromotoren

Die DNA von I19, welche nicht durch das Enzym Sau3A fragmentiert werden kann, wurde mittels des alternativen Vielschneiderenzyms TaqI zu Fragmentlängen kleiner als 1,5 kb partiell gespalten und mit dem ClaI geschnittenen Suchvektor pGL703 ligiert. Der Ligationsansatz wurde direkt als auch indirekt über E. coli nach S. lividans TK23 transformiert. Nach der direkten Transformation und Überschichtung der Regenerationsplatten mit einer Gentamicinkonzentration von 30 $\mu$g/ml im Medium konnten drei Gentamicin-resistente TK23 Kolonien isoliert werden, während nach vorheriger Vermehrung der Hybridplasmide im Ligationsgemisch in E. coli mit nachfolgender DNA-Isolierung und Transformation nach S. lividans TK23 dreizehn Gentamicin-resistente Einzelkolonien isoliert wurden (indirekter Weg). Alle so erhaltenen Selektanten erwiesen sich im Nachtest wie erwartet als Kanamycinresistent.

Beispiel 3: Klassifizierung der I19 Promotoren

Die Plasmide der Gentamicin-resistenten Streptomycetenkolonien wurden reisoliert, nach E. coli transformiert und hier bezüglich der Insertionen genauer charakterisiert. Dabei zeigte sich, daß der Großteil der isolierten promotortragenden DNA-Fragmente auch in E. coli aktiv ist, d.h. zu einer Expression des Indikatorgens führt. Daneben wurden auch Fragmente isoliert, die nur in Streptomyceten eine Transkriptionsaktivität aufweisen. Anhand dieser Eigenschaften und auch auf Basis der von den Promotoren aus vermittelte Gentamicin-Resistenzhöhe sowohl in Streptomyceten als auch in E. coli (im Fall der SEP-Sequenzen) wurden die isolierten I19 Promotoren klassifiziert. Die stärkste Gentamicinresistenzgen-Expressionsrate in beiden Organismen zeigte der Promotor p14 als SEP-Sequenz, in S. lividans der reine Streptomyceten-Promotor pS1. Die vergleichsweise niedrige Resistenzhöhe von 5-10 $\mu$g Gm/ml liegt in der relativen schwachen Translationsrate des Indikatorgens in Streptomyceten begründet. Daher ist dieses Suchsystem besonders für die Isolierung stärkerer Promotoren aus Streptomyces oder deren Bakteriophagen geeignet. Eine genauere Charakterisierung der Promotorstärken wurde auf m-RNA Ebene vorgenommen. Dadurch konnte die primär vorgenommene Klassifizierung verifiziert werden. Dot-Blot-Analysen, in denen die Ausbeute an Nm-m-RNA als interne Kontrolle diente, bestätigten, daß p14 die stärkste Transkriptionsaktivität aller identifizierten I19 Promotoren in S. lividans aufweist.

Beispiel 4: Subklonierung der Promotoren zum Nachweis ihrer Aktivität außerhalb des Suchvektors

Um Artefakte, wie z.B. Sequenzkonstellationen aus der mehrfachen Klonierungsstelle von pGL703 und dem inserierten DNA-Fragment, die zu einer Promotoraktivität führen könnten, auszuschließen, wurden p14 und pS1 in den Vektor pIJ487 (erhältlich bei der John Innes Foundation, Norwich, England) subkloniert und in S. lividans auf die Expression des promotorlosen Neomycingens selektioniert. In anschließenden Nm-MIC-Tests zeigte sich, daß p14 zu einer doppelt so hohen, pS1 in diesem System zu etwa einem Viertel der Neomycinresistenz führt wie der erm-up Promotor, der bislang in der Literatur zu den stärksten Streptomycetenpromotoren gezählt wird. Diese Ergebnisse bestätigen nochmals die Eignung des neuen Suchvektors zur Isolierung besonders starker Streptomycetenpromotoren.

Legende zu Fig. 1: Promotorsuchvektor pGL703

"mcs" steht für "Multiple Clonierungsstelle" und beinhaltet Spaltstellen für die Restriktionsenzyme

EcoRI SstI KpnI SmaI PstI HindIII ClaI XbaI BamHI EcoRI SstI KpnI

Gm$\Delta$P bedeutet promotorloses Gentamicin[R]-Gen; Cm[R] bedeutet Resistenzgen gegen Chloramphenicol; Nm[R] bedeutet Resistenzgen gegen Neomcycin; fd steht für den Transkriptionsterminator des Phagen fd.

Legende zu Fig. 2: Restriktionskarte der I19 Phagen-DNA

Oben sind die Schnittstellen der einzelnen Restriktionsenzyme dargestellt, unten die Länge und Position der einzelnen Restriktionsschnittstücke für jedes der verwendeten 6 Enzyme.

Legende zu Fig. 3: Identifizierte Promotor-Regionen auf dem Genom von I19

Die Promotorregionen sind in der Genomkarte von I19 als I und II eingetragen.

Tabelle 1: DNA-Sequenz des Promotors p14

```
TCGAATCAGCCGGATTCGCGGAAGACGTACAGGTGCACTGGAAGCCTGTAGAGACCTTCG
AGCTTAGTCGGCCTAAGCGCCTTCTGCATGTCCACGTGACCTTCGGACATCTCTGGAAGC
^  ^      ^    ^     ^     ^     ^ ^       ^                           ^
```

1 TAQI, 3 HINFI, 10 HPAII, 13 HINFI, 17 FNUDII, 21 MBOII, 25
 MAEII, 27 RSAI, 33 HGIAI SDUI SDUI, 58 TAQI,

```
ATGGATGAGCAATCGAGAAGTAAGCACACCGGGCGGATTTCCGCCAAGCTTCCTATCCAG
TACCTACTCGTTAGCTCTTCATTCGTGTGGCCCGCCTAAAGGCGGTTCGAAGGATAGGTC
      ^         ^              ^                 ^^        ^
```

63 FOKI, 73 TAQI, 89 HPAII NCII SCRFI, 106 HINDIII, 107 ALUI
, 117 APYI ECORII SCRFI,

```
                                        1
GAGATATTATGAGTTACGTAGACCTACGCCTTGACCTTGATGAGGCGGCGTGAGCTACAA
CTCTATAATACTCAATGCATCTGGATGCGGAACTGGAACTACTCCGCCGCACTCGATGTT
          ^  ^^  ^             - 35 II  - 35 I ^   ^        ^  - 10 II
```

133 MAEIII, 135 SNABI, 136 MAEII, 138 ACCI, 162 MNLI, 165 FN
UIVHI, 173 ALUI,

```
        1                         2     1                      1
TCAATACTCGATTAGGTCAAGGTGGAACGCAGAGAGGGTCTGACTGCCTGAGTCGGTAGT
AGTTATGAGCTAATCCAGTTCCACCTTGCGTCTCTCCCAGACTGACGGACTCAGCCATCA
 - 10 I    ^               RBSI          ^           ^  ^
```

188 TAQI, 214 MNLI, 228 DDEI, 230 HINFI,

```
                                    2        1
CAGGTGATGAGGGAGATAGAGCCAAGCAAAGAGGAGAGGGTCATTGCGGGTTAGTGCTAC
GTCCACTACTCCCTCTATCTCGGTTCGTTTCTCCTCTCCCAGTAACGCCCAATCACGATG
    ^      ^           ^        RBS^II    ^
```

243 HPHI, 249 MNLI, 263 TTH111II, 271 MNLI, 276 MNLI,

```
TCGATGTACCTGGAGAGGAGTTCCCCAAACTCCGCCTTCTCGCCCTCTGTCAGGTCGA
AGCTACATGGACCTCTCCTCAAGGGGTTTGAGGCGGAAGAGCGGGAGACAGTCCAGCT
^     ^    ^^      ^                                  ^            ^
```

301 TAQI, 306 RSAI, 309 APYI ECORII SCRFI, 310 GSUI, 315 MNL
I, 344 MNLI, 355 TAQI,

Tabelle 2:DNA-Sequenz des Promotors pS1

```
TCGAGGTAAATACCTCTTCGGCTAGTCCTTCGTAATAGTCTTCTGCGGTTGTGTAATCGT
AGCTCCATTTATGGAGAAGCCGATCAGGAAGCATTATCAGAAGACGCCAACACATTAGCA
^  ^         ^  ^        ^                      ^
```

1 TAQI, 3 MNLI, 13 MNLI, 15 MBOII, 22 MAEI, 39 MBOII,

```
CTCTCCTATCGAGCTGCCATCGCGCTCCGCAGATGACGCAGAACAGCTCTGCTCTAGATG
GAGAGGATAGCTCGACGGTAGCGCGAGGCGTCTACTGCGTCTTGTCGAGACGAGATCTAC
        ^  ^^        ^^          ^          ^        ^^
```

69 TAQI, 72 ALUI, 73 BBVI FNUIVHI, 81 FNUDII, 82 HHAI, 95 HG
AI, 105 ALUI, 113 XBAI, 114 MAEI,

```
TTATCAGAGTACACTCGGTCCACGAATACCCGGCCGGTGGACTTATTACAGCGCATTGAC
AATAGTCTCATGTGAGCCAGGTGCTTATGGGCCGGCCACCTGAATAATGTCGCGTAACTG
         ^      ^ ^          ^^^^ ^              ^        ^
```

129 RSAI, 135 TAQII, 137 ASUI AVAII, 149 NCII SCRFI, 150 HPA
II, 151 CFRI GDIII XMAIII, 152 HAEIII, 154 HPAII, 171 HHAI,
178 ACYI HGAI,

```
            1'                                1       -'351'
GCCACCCTTATAGGTAACGTCGGTGACCGCCGAAGCGTGCCAGAGCTACCCGCC|TTGTAC|
CGGTGGGAATATCCATTGCAGCCACTGGCGG|CTT|CGCACGGTCTCGATGGGCGG|AACATG|
  ^         ^    ^    ^^      2'         ^              ^
```

184 HGIEII, 194 MAEIII, 197 MAEII, 202 BSTEII HPHI, 203 MAEI
II, 224 ALUI, 237 RSAI,

```
         -'101'            1            -'101'  1'
GAGGCCAGGGACAG|CAGAAG|CGAAAGCTACCGCTGCACCAC|CAGAAG|TACCGAAGAAACC
CTCCGGTCCCTGTC|GTCTT|GCTTTCGATGGCGACGTGGTG|GTCTTC|ATGGCTTCTTTGG
^^^ ^          2'      ^       ^ ^          2'    ^ 2'       ^
```

241 MNLI, 242 HAEI, 243 HAEIII, 245 APYI ECORII SCRFI, 265 A
LUI, 270 NSPBII, 272 BBVI FNUIVHI, 287 RSAI, 292 MBOII, 299
HPAII,

```
                                           1'
GGACCAATCAAAGTCGAGAGCCTAGCGGCCCTCAGTTCCTTCTTCTCGGATACCCGGCGA
CCTGGTTAGTTTCAGCTCTCGGATCGCCGGGAGTCAAGGAAGAAGAGCCTATGGGCCGCT
^              ^      ^   ^ ^  ^^          ^          ^^
```

301 ASUI AVAII, 314 TAQI, 322 MAEI, 325 FNUIVHI, 327 ASUI HA

EIII, 330 MNLI, 331 DDEI, 341 MBOII, 353 NCII SCRFI, 354 HPA II,

-35 II'    1'                    -10 II'

CAGATGACCTTTGCCGGTACCCCATCAAGGATTGAGAACCAGGCGTCACCACCTTGATTA
GTCTACTGGAAACGGCCATGGGGTAGTTCCTAACTCTTGGTCCGCAGTGGTGGAACTAAT

RBS

367 HGIEII, 374 HPAII, 376 HGICI KPNI NLAIV, 377 RSAI, 399 A
PYI ECORII SCRFI, 402 ACYI, 403 HGAI, 405 MAEIII, 406 HPHI,

CTTTCGA
GAAAGCT

424 TAQI,

## Patentansprüche

1. Promotorsuchvektor, replizierbar in E. coli und Streptomyceten, enthaltend
   (a) einen Selektionsmarker für Streptomyceten
   (b) einen Selektionsmarker für E. coli
   (c) als Indikatorgen ein promotorloses Gentamicinresistenzgen
   (d) einen Terminator stromaufwärts vom Indikatorgen
   (e) sowie eine mehrfache Klonierungsstelle stromaufwärts vom Indikatorgen.

2. Promotorsuchvektor nach Anspruch 1, dadurch gekennzeichnet, daß es das Plasmid pGL703 ist.

3. Promotorsuchvektor nach Anspruch 1, dadurch gekennzeichnet, daß das Indikatorgen im 5'-Kodierbereich zwei oder mehr TTA-Kodons besitzt.

4. Verfahren zur Isolierung von Promotoren, dadurch gekennzeichnet, daß möglicherweise Promotoren enthaltende DNA-Fragmente vor das Indikatorgen von Promotorsuchvektoren nach Anspruch 1, 2 oder 3 einligiert werden und die Promotoraktivität über die Expressionsrate des Indikatorgens bestimmt wird.

5. Promotor p14, enthaltend promotoraktive Sequenzen aus Tabelle 1.

6. Promotor pS1, enthaltend promotoraktive Sequenzen aus Tabelle 2.

7. Verwendung der Promotoren nach Anspruch 5 oder Anspruch 6 zur Expression von Fremdproteinen in E. coli oder Streptomyces.

## Patentansprüche für folgende Vertragsstaaten : ES, GR

1. Verfahren zur Isolierung von Promotoren, dadurch gekennzeichnet, daß ein möglicherweise Promotoren enthaltendes DNA-Fragment vor das Indikatorgen eines Promotorsuchvektors einligiert wird, wobei der Promotorsuchvektor in E. coli und Streptomyceten replizierbar ist und
   (a) einen Selektionsmarker für Streptomyceten,
   (b) einen Selektionsmarker für E. coli,
   (c) als Indikatorgen ein promotorloses Gentamicinresistenz-Gen,
   (d) einen Terminator stromaufwärts vom Indikatorgen,
   (e) sowie eine mehrfache Klonierungsstelle stromaufwärts vom Indikatorgen enthält.

**2.** Verfahren nach Anspruch 1, dadurch gekennzeichnet, daß als Suchvektor das Plasmid pGL703 eingesetzt wird.

**3.** Verfahren zur Expression von Fremdproteinen in E. coli oder Streptomyces, dadurch gekennzeichnet, daß ein nach dem Verfahren von Anspruch 1 oder 2 gewonnener Promotor eingesetzt wird.

**4.** Verfahren nach Anspruch 3, dadurch gekennzeichnet, daß der Promotor p14 oder der Promotor pS1 eingesetzt wird.

Fig. 1

mcs:  EcoRI  SstI  KpnI  SmaI  PstI  HindIII  ClaI  XbaI  BamHI  EcoRI  SstI  KpnI

EP 0 531 717 A2

Fig. 2

BstEII
ClaI
HaeII
BstEII

BstEII
HaeII
AccI
HaeII

AccI
HaeII

AccI
BstEII

HpaI

HaeII

HaeII

EcoRI
BstEII

0.0 kb

13.6 kb

*Fig. 3*